# EUROPEAN PATENT APPLICATION

(11) **EP 1 078 645 A2**
(43) Date of publication of application: **28.02.2001**
(21) Application number: 00305623.1
(22) Date of filing: 04.07.2000
(51) Int. Cl.: A61M 16/04, A61M 39/10

(54) **Medico-surgical tube assemblies**

(30) Priority: 20.08.1999 GB 9919652
(71) Applicant: Smiths Industries Public Limited Company, London, NW11 8DS (GB)
(72) Inventor: Pagan, Eric, Hythe, Kent CT21 6DN (GB); Watton, Joan Elizabeth, Hythe, Kent CT21 6DW (GB); Solly, Caroline Therese, Ashford, Kent TN23 5DE (GB)
(74) Representative: Flint, Jonathan McNeill

(57) **Abstract**

Connection is made to an endotracheal tube 1, which is helically reinforced up to its machine end 11, by means of a connector 2. The connector 2 has, at its rear end 21, a tapered external surface 29 and, at its forward end 20, has an external screw thread 25, the profile of which is shallow on its forward edge 26 and steeper on its rear edge 28. The pitch of the thread 25 is the same as the pitch of the reinforcement 15 in the tube 1 so that the connector 2 can be screwed into the tube.

## Description

This invention relates to medico-surgical tube assemblies of the kind comprising a medico-surgical tube having a reinforcement element extending helically along the tube, and a connector having a forward portion inserted in the tube.

Medico-surgical tubes, such as tracheal tubes, are commonly reinforced by incorporating a helical reinforcing element, such as a metal wire, within the wall of the tube. Such a tube can be more resistant to crushing but can still be flexible. One problem with such tubes, however, is that the reinforcing element can prevent connection being made to the tube by a conventional tapered male connector. To overcome this problem, an unreinforced portion is often formed at the machine end of the tube. This can be done by removing the reinforcement and smoothing the damage this causes, or by attaching a separate unreinforced section to the machine end of the tube. Proposals have been made for improving conventional connectors for use with unreinforced tubes or tube sections, as described in WO98/24500 in which a screw thread on the outside of the connector is shaped to enable the connector to be pushed in.

It is an object of the present invention to provide an assembly of a helically-reinforced tube and a connector, which does not require the provision of an unreinforced section at the machine end of the tube.

According to one aspect of the present invention there is provided an assembly of the above-specified kind, characterised in that the connector has a thread formation on the outer surface of its forward portion, that the tube is reinforced substantially to its machine end, and that the thread formation on the connector cooperates with the reinforcement element so that the connector can be screwed into the end of the tube

The connector preferably has an externally-projecting sealing ring towards the forward end of the thread formation. The thread formation may be a continuous screw thread and preferably has the same pitch as that of the reinforcing element. The thread formation on the connector preferably has a shallow forward edge and a steeper rear edge. The connector may have a flange between the forward portion and a rear portion. The connector may have a rear portion with a taper on its external surface for making connection to a female tapered coupling. The internal surface of the tube is preferably substantially smooth.

According to another aspect of the present invention there is provided a method of making connection to a helically-reinforced medico-surgical tube comprising the steps of providing the tube reinforced substantially to its machine end, providing a connector with a thread formation on its external surface towards its forward end, and screwing the forward end of the connector into the machine end of the tube so that the thread formation aligns between pitches of the reinforcement element and draws the connector into the tube.

The method may include the step of cutting a length from the machine end of the tube prior to connecting the connector.

An endotracheal tube assembly and a method of making connection to an endotracheal tube, according to the present invention, will now be described, by way of example, with reference to the accompanying drawings, in which:
- Figure 1: is a side elevation view of the assembly;
- Figure 2: is an enlarged sectional side elevation view of the machine end of the assembly;
- Figure 3: is an enlarged part-sectional, side elevation view of the connector; and
- Figure 4: is an enlarged side elevation view of an alternative connector.

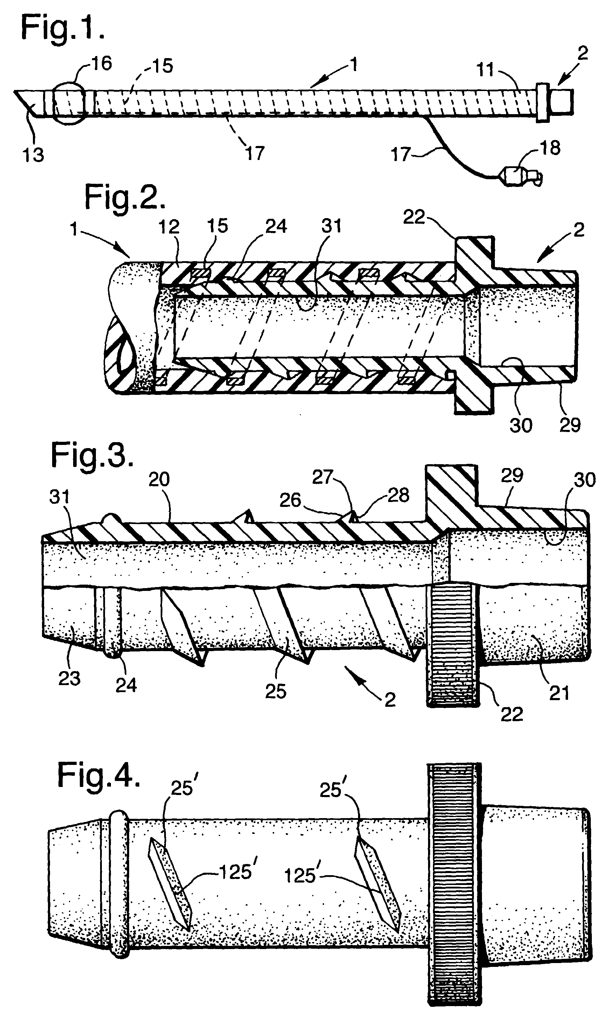

With reference first to Figures 1 to 3, the assembly comprises an endotracheal tube 1 and a connector 2 inserted in the rear, machine end 11 of the tube.

The endotracheal tube 1 has an extruded wall 12 of a relatively soft, flexible thermoplastics material such as PVC, polyurethane or nylon. Other materials, such as rubber, could be used; other processes, such as dipping, could be used to form the tube. The tube 1 has a circular section with an internal diameter of 5mm and is typically about 300 mm long and has a naturally straight shape. The patient end 13 of the tube is bevelled to one side.

The tube includes a reinforcing element 15 extending from a location just rearwardly of the bevel on the patient end 13 to the machine end 11. The reinforcing element 15 is of a relatively hard material such as a PVC, polyester or nylon filament of rectangular section and about 0.25 mm thick wound into a helical path with a pitch of 1.4mm. Alternatively, the filament 15 could have a circular section, although the rectangular section helps reduce the wall thickness. The filament 15 could be made of other materials, such as metal, polyester, nylon, Kevlar, polyethylene or polypropylene. The filament 15 is embedded in the wall 12 so that the internal and external surfaces of the tube are substantially smooth. The tube has an optional inflatable cuff 16 close to its patient end 13 connected via an inflation lumen 17 to a pilot balloon and connector 18.

The connector 2 may be of the kind described in WO98/24500. The connector 2 is moulded of a rigid plastics material, such as polycarbonate, nylon or the like. The connector is divided into a forward portion 20 and a rear portion 21 by a radially-extending flange and finger grip 22. The forward end 23 of the forward portion 21 is tapered or rounded on its outer surface or edge to form a reduced diameter leading edge. Alternatively, or additionally, the forward end of the connector could be bevelled. Just to the rear of the tapered end 23, there is an annular sealing ring 24 projecting from the outside of the connector. The sealing ring 24 has a semi-circular section or some other shape. A continuous screw thread formation 25 extends helically around the forward portion 20 from a point just to the rear of the sealing ring 24 up to the flange 22. The thread formation 25 projects outwardly of the connector 2 and forms a single start screw thread with a pitch of 1.4mm. The height of the thread 25 is greater than that of the sealing ring 24. Preferably, the thread 25 has a shallow forward sloping edge 26, a relatively sharp peak 27 and a steeper rear edge 28, since this has been found to give an improved resistance to an axial force applied to pull the connector 2 out of the tube 1. However, a conventional symmetric screw thread could be used. The external diameter of the forward portion 20 is constant along its length rearwardly of the tapered forward end 23.

The curved outer edge of the flange 22 is knurled or otherwise shaped to improve grip on the connector 2 during rotation, for example, the flange could have a non-circular shape.

The rear portion 21 of the connector 2 has a conventional 8.5mm, 15mm or 22mm male taper on its external surface 29 adapted to receive a conventional female, tapered connector of cooperating size, not shown. Alternatively, the machine end of the connector could be shaped to receive other forms of connector such as a screw-threaded connector. A recess 30 within the rear portion 21 opens to a straight, axial bore 31 extending through the forward portion 20.

Connection is made to the machine end 11 of the tube 1 by pushing the forward end 23 of the connector 2 into the machine end of the tube, guided by the taper. The connector 2 is pushed in beyond the sealing ring 24 until the thread 25 starts to enter the tube 1. The resilience of the material of the wall 12 enables it to be compressed slightly against the inside surface of the reinforcement filament 15, which is relatively inelastic radially. This resilience of the wall 12 is sufficient to allow entry of the sealing ring 24. Further entry of the connector 2 by simple axial movement is prevented because the material of the wall 12 cannot be deformed sufficiently to accommodate the higher section of the thread 25. When this resistance to further axial movement is felt, the user twists the connector 2 clockwise or while applying axial force so that the thread formation 25 on the connector, which has the same pitch as that of the reinforcement filament 15, screws into the reinforced tube 1 and aligns in the space between turns of the filament where the tube wall 12 can be deformed radially to a greater extent. The connector 2 is screwed in until the machine end 11 of the tube 1 contacts the forward side of the flange 22. It will be appreciated that if the reinforcement filament is wound helically in the opposite sense, the thread formation on the connector would be of the same sense and the connector would be inserted by screwing with an anticlockwise rotation.

After use, the entire assembly may be disposed of together, or the connector 2 could be unscrewed from the tube 1 for reuse.

The assembly provides high security against accidental disconnection of the connector from the tube because they can only be disconnected by unscrewing.

The connector 2 enables connection to be made to helically-reinforced tubes 1 without the need to provide an unreinforced portion. This, therefore, simplifies and reduces the cost of manufacture of the tube.

The present invention also enables cut-to-length reinforced tubes to be provided. It can be advantageous in some applications to have a tube that is initially relatively long and that is then cut close to the patient since this minimizes the length of protruding tube. For example, where a tracheal tube is inserted to replace a laryngeal mask airway LMA, this can be done by inserting the tracheal tube through the LMA and then withdrawing the LMA over the tracheal tube, which is left in place. Initially, the tracheal tube should be relatively long to enable withdrawal of the LMA, but it should then, preferably be shortened by cutting close to the patient's mouth. Previously, it has been difficult to cut reinforced tubes to length because this could result in loss of the unreinforced section needed to make connection.

It will be appreciated that there are various modifications that could be made to the connector and tube. In some assemblies, the sealing ring at the forward end of the connector may not be necessary. The thread formation need not be a continuous screw thread extending between every turn of the reinforcement filament. Instead, as shown in Figure 4, the thread formation 25 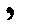 it could be a series of short, angled projections 125 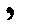 forming an interrupted, broken helical path where the projections only lie between alternate turns of the helical reinforcement.

The invention is not confined to tracheal tube assemblies but could be used in other medico-surgical tube assemblies

## Claims

1. An assembly comprising a medico-surgical tube (1) having a reinforcement element (15) extending helically along the tube, and a connector (2) having a forward portion (20) inserted in the tube, characterised in that the connector (2) has a thread formation (25, 25') on the outer surface of its forward portion (20), that the tube (1) is reinforced substantially to its machine end (11), and that the thread formation (25, 25') on the connector cooperates with the reinforcement element (15) so that the connector (2) can be screwed into the end (11) of the tube (1).

2. An assembly according to Claim 1, characterised in that the connector (2) has an externally-projecting sealing ring (24) towards the forward end of the thread formation (25, 25').

3. An assembly according to Claim 1 or 2, characterised in that the thread formation is a continuous screw thread (25).

4. An assembly according to any one of the preceding claims, characterised in that the thread formation (25, 25') has the same pitch as that of the reinforcement element (11).

5. An assembly according to any one of the preceding claims, characterised in that the thread formation (25, 25') on the connector (2) has a shallow forward edge (26) and a steeper rear edge (28).

6. An assembly according to any one of the preceding claims, characterised in that the connector (2) has a flange (22) between the forward portion (20) and a rear portion (21).

7. An assembly according to any one of the preceding claims, characterised in that the connector (2) has a rear portion (21) with a taper on its external surface (29) for making connection to a female tapered coupling.

8. An assembly according to any one of the preceding claims, characterised in that the internal surface of the tube (1) is substantially smooth.

9. A method of making connection to a helically-reinforced medico-surgical tube (1) comprising the steps of providing the tube (1) reinforced substantially to its machine end (11), providing a connector (2) with a thread formation (25, 25') on its external surface towards its forward end, screwing the forward end of the connector (2) into the machine end (11) of the tube (1) so that the thread formation (25, 25') aligns between pitches of the reinforcement element (15) and draws the connector (2) into the tube (1).

10. A method according to Claim 9, including the step of cutting a length from the machine end of the tube (11) prior to connecting the connector (2).
